Europäisches Patentamt

European Patent Office

Office européen des brevets

(11) Veröffentlichungsnummer: **0 262 239 B1**

(12) **EUROPÄISCHE PATENTSCHRIFT**

(45) Veröffentlichungstag der Patentschrift: **30.12.92**

(21) Anmeldenummer: **86113343.7**

(22) Anmeldetag: **29.09.86**

(51) Int. Cl.⁵: **A61M 16/20**

(54) **Atemvorrichtung.**

(43) Veröffentlichungstag der Anmeldung:
**06.04.88 Patentblatt 88/14**

(45) Bekanntmachung des Hinweises auf die
Patenterteilung:
**30.12.92 Patentblatt 92/53**

(84) Benannte Vertragsstaaten:
**AT BE CH DE FR GB IT LI LU NL SE**

(56) Entgegenhaltungen:
**FR-A- 2 573 658**
**GB-A- 2 019 222**
**US-A- 4 111 228**
**US-A- 4 210 174**

(73) Patentinhaber: **Brugger, Stephan,**
**Dipl.-Wirt.-Ing.**
**Etztalstrasse 21**
**W-8137 Berg(DE)**

(72) Erfinder: **Der Erfinder hat auf seine Nennung**
**verzichtet**

(74) Vertreter: **Hoffmann, Klaus, Dr. rer. nat. et al**
**Hoffmann . Eitle & Partner Patentanwälte**
**Postfach 81 04 20 Arabellastrasse 4**
**W-8000 München 81(DE)**

**Beschreibung**

Die Erfindung betrifft eine Atemvorrichtung zum kontrollierten Ein- und Ausatmen zwecks Therapie der Atemwege, umfassend einen Einlaßstutzen für einzuatmende Luft, einen Auslaßstutzen für ausgeatmete Luft, einen Verbindungsstutzen, der mit dem Einlaßstutzen und dem Auslaßstutzen in Verbindung steht, ein im Einlaßstutzen vorgesehenes, als Einwegventil ausgebildetes Einlaßventil, das in Richtung der Strömung im Einlaßstutzen öffnet und in Gegenrichtung sperrt, einen am Auslaßstutzen angeordneten, in der Fachwelt auch als Stenose bezeichneten Ausatemwiderstand, gegen den der Patient ausatmet, und ein im Strömungsweg des Auslaßstutzens angeordnetes, als Einwegventil ausgebildetes Auslaßventil, das in Richtung der Strömung im Auslaßstutzen öffnet und in Gegenrichtung sperrt, wodurch ein Einatmen von Luft durch den Ausatemwiderstand und den Auslaßstutzen verhindert wird.

Eine Atemvorrichtung dieser Art ist aus der Druckschrift US-A-4 210 174 bekannt. Bei der dort in Fig. 5 dargestellten Ausführungsform ist das Auslaßventil im Auslaßstutzen angeordnet und der Ausatemwiderstand in Form eines kompliziert aufgebauten Kegelventils ausgebildet.

Da beim therapeutischen Einsatz von Atemvorrichtungen der eingangs genannten Art zwangsläufig Sputum des Patienten zum Auslaßventil und den Ausatemwiderstand gelangt, müssen diese Bauteile zur Vermeidung der Übertragung von Krankheiten vor jedem Patientenwechsel sorgfältig gereinigt und desinfiziert werden, wozu diese Bauelemente aus bzw. von der Vorrichtung entfernt werden müssen. Bei der vorbekannten Vorrichtung muß der Ausatemwiderstand aufgrund seiner komplizierten Konstruktion, welche Nischen und sonstige, von einer Spülung schwer erfaßbare Innenräume bildet, zur Reinigung und Desinfizierung praktisch zerlegt und anschließend wieder sorgfältig zusammengesetzt werden, was einen erheblichen Einsatz von entsprechend geschultem Laborpersonal bedingt.

Aus der Druckschrift US-A-4 111 228 ist eine Atemvorrichtung für die Anästhesie bekannt, bei welcher in einem Einlaßstutzen ein Einlaßventil und in einem Auslaßstutzen ein Auslaßventil vorgesehen sind. Ein Ausatemwiderstand ist bei dieser Vorrichtung nicht vorhanden.

Der Erfindung liegt die Aufgabe zugrunde, eine Atemvorrichtung der eingangs genannten Art zu schaffen, bei welcher das Auslaßventil und der Ausatemwiderstand vor jedem Patientenwechsel mit geringem personellem Aufwand gereinigt und desinfiziert werden können.

Erfindungsgemäß wird diese Aufgabe mit den Merkmalen des kennzeichnenden Teils des Patentanspruchs 1 gelöst.

Die erfindungsgemäße Vorrichtung weist den Vorteil auf, daß sie einen überraschend einfachen Aufbau besitzt und die Reinigung und Desinfektion von Auslaßventil und Atemwiderstand auf einfache und zeitsparende Weise ermöglicht.

Weiterbildungen der Erfindung ergeben sich aus den dem Patentanspruch 1 nachgeordneten Unteransprüchen.

An den Einlaßstutzen der erfindungsgemäßen Vorrichtung läßt sich problemlos ein medizin-technisches Gerät, beispielsweise ein Aerosol-Vernebler, anschließen. Dabei kann gewährleistet werden, daß die vom Patienten eingeatmete Luft ausschließlich aus dem Vernebelungsgerät stammt und keine Nebenluft unkontrolliert zuströmt. Dabei können gleichzeitig, d. h. während einer einzigen Behandlungssitzung, sowohl eine Atemtherapie als auch eine atemunterstützende Inhalationsbehandlung durchgeführt werden.

Vorteilhaft sind das Einlaßventil und das Auslaßventil demontierbar ausgebildet. Es ist deshalb leicht möglich, das Gerät in seine Einzelbestandteile zu zerlegen, um es zu reinigen und zu desinfizieren.

Vorteilhaft bestehen Auslaßventil und Einlaßventil jeweils aus einem Ringeinsatz und einer gegen die Stirnseite dieses Ringeinsatzes abdichtenden, einseitig angelenkten Klappe. Bevorzugt besteht die Klappe aus flexiblem, insbesondere gummielastischem Material. Die Klappe kann auf einfachste Weise mittels einer oder mehrerer, vorzugsweise zwei Stiften auf dem Ringeinsatz befestigt sein. Bei sehr hohen Ansprüchen an die erzielbare Dichtigkeit der Ventile kann eine zusätzliche Dichtlippe zwischen Ringeinsatz und Klappe vorgesehen sein. Solche, als separate Baueinheit ausgebildete Einwegventile lassen sich fertigungstechnisch leicht und in großen Stückzahlen herstellen und sind auf einfachste Weise in die Atemvorrichtung einsetzbar. Zu Reinigungs- und Desinfektionszwecken oder im Falle eines Defektes lassen sie sich leicht austauschen.

In zweckmäßiger Weiterbildung der Erfindung ist der Ausatemwiderstand auf den Auslaßstutzen aufsteckbar. Das Auslaßventil ist dann zweckmäßigerweise in das freie Ende des Auslaßstutzens eingesetzt. Nach Abziehen des aufgesteckten Ausatemwiderstands vom Auslaßstutzen wird so das Auslaßventil frei zugänglich und läßt sich leicht herausnehmen. Nach dem Wiederaufstecken des Ausatemwiderstands ist es fest in seiner vorgesehenen Lage fixiert.

Das Einlaßventil ist vorzugsweise in das freie Ende des Einlaßstutzens eingesetzt; das beispielsweise lediglich klemmend befestigte Einlaßventil läßt sich dort ebenfalls gut demontieren.

Bei der erfindungsgemäßen Atemvorrichtung ist der Ausatemwiderstand als abgestuftes Rohr ausgebildet, wobei der Innendurchmesser des auslaßseitigen Rohrabschnitts wesentlich kleiner ist als derjenige des Auslaßstutzens. Die sich hierdurch ergebende Querschnittsverengung in Richtung des Luftauslasses ergibt den gewünschten Strömungswiderstand, gegen den der Patient ausatmen muß. Das abgestufte Rohr läßt sich gut auf das Ende des Auslaßstutzens aufstecken; der Strömungswiderstand ist durch die Wahl des Innendurchmessers des auslaßseitigen Rohrabschnitts wählbar. Der Ausatemwiderstand kann aber auch mittels einstellbarer Ausblasöffnungen, durch welche die ausgeatmete Luft ins Freie gelangt, gebildet werden.

Besonders günstige Strömungsverhältnisse ergeben sich, wenn Einlaßstutzen und Auslaßstutzen gleichsinnig schräg in den Verbindungsstutzen einmünden. Einlaßstutzen und Auslaßstutzen bilden dabei einen spitzen Winkel. An keiner Stelle muß die strömende Luft mehr als unbedingt notwendig umgelenkt werden, wodurch nicht nur zusätzliche Strömungswiderstände vermieden werden, sondern auch die Gefahr der inneren Verschmutzung des Gerätes wesentlich herabgesetzt ist. Vorzugsweise beträgt der Winkel zwischen Einlaßstutzen und Auslaßstutzen zwischen 30 und 50 Winkelgraden.

Besonders vorteilhaft ist eine Ausführungsform, bei der auf den Verbindunsstutzen ein Mundstück aufgesteckt ist. Dieses Mundstück läßt sich ebenfalls leicht abziehen, so daß sich das gesamte Gerät in seine Einzelteile zerlegen und gut desinfizieren läßt. Anstelle des Mundstücks kann auch der Adapteranschluß einer aufblasbaren Atemmaske an den Verbindungsstutzen angesteckt werden.

Der mögliche Anschluß eines medizintechnischen Geräts, insbesondere eines Aerosol-Vernebelungsgerätes, wird erleichtert, wenn der Einlaßstutzen an seinem freien Ende einen speziell dafür vorgesehenen, lösbaren Anschluß aufweist. Eine mit einem solchen zusätzlichen Anschluß ausgerüstete Atemvorrichtung ist besonders für die Durchführung der oben beschriebenen Doppeltherapie prädestiniert.

Schließlich kann eine verschließbare Öffnung vorgesehen sein, an welche eine Druckmeßvorrichtung anschließbar ist. Auf diese Weise wird eine Kontrolle des vom Patienten aufgebrachten Drucks beim Ausatmen während der Therapie ermöglicht.

Ein Ausführungsbeispiel der Erfindung wird nachstehend anhand der beigefügten Zeichnungen näher erläutert. Es zeigen:

Fig. 1: eine Atemvorrichtung gemäß der Erfindung, mit aufgestecktem, rohrabschnittsförmigen Ausatemwiderstand, aufgestecktem Mundstück und an den Einlaßstutzen angeschlossenem Aerosol-Vernebler, in einem Schnittbild; und

Fig. 2: das in die Atemvorrichtung gemäß Fig. 1 eingesetzte Auslaßventil, in einer vergrößerten perspektivischen Darstellung.

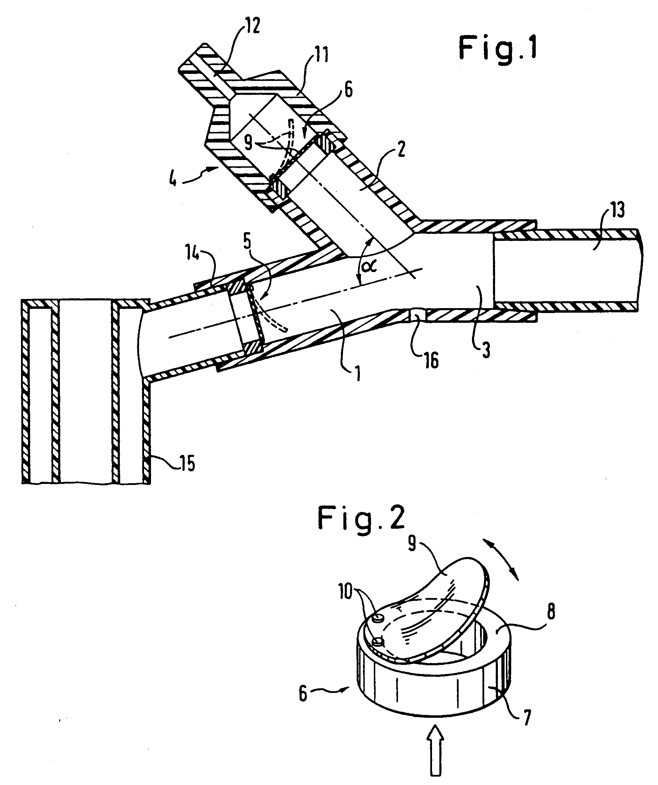

Die in Fig. 1 dargestellte Atemvorrichtung besitzt einen Einlaßstutzen (1) für einzuatmende Luft, einen Auslaßstutzen (2) für ausgeatmete Luft sowie einen Verbindungsstutzen (3), der mit den Atemwegen des Patienten in Verbindung steht. Einlaßstutzen (1) und Auslaßstutzen (2) münden schräg in den Verbindungsstutzen (3) ein, wobei sie einen Winkel von ungefähr 60 Winkelgraden einschließen. Auf das Ende des Auslaßstutzens (2) ist ein Ausatemwiderstand (4) aufgesteckt. Am freien Ende des Einlaßstutzens (1) ist ein als Einwegventil ausgebildetes Einlaßventil (5) eingesetzt. Ein Einwegventil gleicher Konstruktion befindet sich als Auslaßventil (6) im Auslaßstutzen (2) vor dem Ausatemwiderstand (4).

In Fig. 2 ist das Auslaßventil (6) herausgezeichnet. Es besteht aus einem Ringeinsatz (7), auf dessen Stirnseite (8) eine Klappe (9) einseitig angelenkt ist. Die Klappe (9) besteht aus gummielastischem Material und ist mittels zweier Stifte (10) auf der Stirnseite (8) des Ringeinsatzes (7) befestigt. Der Ringeinsatz (7) selbst sowie die Stifte (10) bestehen aus Kunststoff. In geschlossenem Zustand dichtet die Klappe (9) luftdicht gegenüber der ebenen Stirnseite (8) ab. Bei Durchströmung in Pfeilrichtung von unten nach oben hebt die flexible Klappe (9) in die in Fig. 2 gezeigte Stellung ab, so daß das Ventil öffnet. - Das Einlaßventil (5) ist von derselben Art wie das beschriebene Auslaßventil (6).

Das Einlaßventil (5) und das Auslaßventil (6) sind beide klemmend innerhalb des Einlaßstutzens (1) bzw. des Auslaßstutzens (2) befestigt (vgl. Fig. 1), so daß sie sich leicht austauschen lassen. Der Ausatmenwiderstand (4) wird gebildet von einem kurzen abgestuften Rohr (11). Der Innendurchmesser des auslaßseitigen Rohrabschnitts (12) des Rohres (11) ist wesentlich kleiner als derjenige des Auslaßstutzens (2). Durch diese Querschnittsverengung ergibt sich der gewünschte Strömungswiderstand, gegen den der Patient ausatmen muß.

In das freie Ende des Verbindungsstutzens (3) ist ein Mundstück (13) eingesetzt. Der Einlaßstutzen (1) weist an seinem freien Ende einen lösbaren Anschluß (14) auf, an den ein Aerosol-Vernebelungsgerät (15) angeschlossen ist. Atmet nun der Patient über das Mundstück (13) ein, so gelangt ausschließlich Luft bzw. ein Gemisch aus Luft und Aerosol vom Aerosol-Vernebelungsgerät (15) durch das geöffnete Einlaßventil (5) in den Einlaßstutzen (1) und von da in den Verbindungsstutzen (3). Das Auslaßventil (6) im Auslaßstutzen (2) ist dagegen geschlossen, so daß keinerlei Nebenluft durch den Ausatemwiderstand (4) eindringen kann. Atmet der Patient anschließend die verbrauchte Luft aus, so kehrt sich die Strömungsrichtung innerhalb der Atemvorrichtung um. Das Einlaßventil (5) schließt augenblicklich, während das Auslaßventil (6) infolge des ein wirkenden Druckes öffnet, so daß die ausgeatmete Luft durch den Ausatemwiderstand (4) ins Freie gelangen kann. Das Aerosol-Vernebelungsgerät (15) wird dabei durch das geschlossene Einlaßventil (5) zuverlässig vor Verschmutzung durch die angefeuchtete und möglicherweise mit Krankheitskeimen beladene Ausatemluft geschützt. Infolge des wechselseitigen Öffnens und Schließens des Einlaßventils (5) bzw. des Auslaßventils (6) stellen sich in jeder Phase genau definierte Strömungsverhältnisse innerhalb des Geräts ein. Der vom Patienten aufgebrachte Druck während des Ausatmens kann durch eine (nicht dargestellte) Druckmeßvorrichtung kontrolliert werden, welche über eine verschließbare Öffnung (16) etwa im Bereich des Übergangs zwischen Einlaßstutzen (1) und Verbindungsstutzen (3) vorgesehen ist.

**Patentansprüche**

1.  Atemvorrichtung zum kontrollierten Ein- und Ausatmen zwecks Therapie der Atemwege, umfassend
    - einen Einlaßstutzen (1) für einzuatmende Luft,
    - einen Auslaßstutzen (2) für ausgeatmete Luft,
    - einen Verbindungsstutzen (3), der mit dem Einlaßstutzen (1) und dem Auslaßstutzen (2) in Verbindung steht,
    - ein im Einlaßstutzen (1) vorgesehenes, als Einwegventil ausgebildetes Einlaßventil (5), das in Richtung der Strömung im Einlaßstutzen (1) öffnet und in Gegenrichtung sperrt,
    - einen am Auslaßstutzen (2) angeordneten Ausatemwiderstand (4), gegen den der Patient ausatmet, und
    - ein im Strömungsweg des Auslaßstutzens (2) angeordnetes, als Einwegventil ausgebildetes Auslaßventil (6), das in Richtung der Strömung im Auslaßstutzen (2) öffnet und in Gegenrichtung sperrt,
    - wodurch ein Einatmen von Luft durch den Ausatemwiderstand (4) und den Auslaßstutzen (2) verhindert wird,
    dadurch **gekennzeichnet, daß**
    - das Auslaßventil (6) am Auslaßstutzen (2) vorgesehen ist und
    - der Ausatemwiderstand (4) als abgestuftes Rohr (11) ausgebildet ist, wobei der Innendurchmesser des auslaßseitigen Rohrabschnittes (12) wesentlich kleiner ist als derjenige des Auslaßstutzens (2).

2.  Atemvorrichtung nach Anspruch 1, dadurch **gekennzeichnet, daß** der Ausatemwiderstand (4) einstellbare Auslaßöffnungen aufweist.

3.  Atemvorrichtung nach Anspruch 1 oder 2, dadurch **gekennzeichnet, daß** der Ausatemwiderstand (4) auf den Auslaßstutzen (2) aufsteckbar ist.

4.  Atemvorrichtung nach einem der vorangehenden Ansprüche, dadurch **gekennzeichnet, daß** das Auslaßventil (6) in das freie Ende des Auslaßstutzens (2) eingesetzt ist.

5.  Atemvorrichtung nach einem der vorangehenden Ansprüche, dadurch **gekennzeichnet, daß** das Einlaßventil (6) in das freie Ende des Einlaßstutzens (1) eingesetzt ist.

6.  Atemvorrichtung nach einem der vorangehenden Ansprüche, dadurch **gekennzeichnet, daß** das Auslaßventil (6) und/oder das Einlaßventil (5) demontierbar sind.

7.  Atemvorrichtung nach einem der vorangehenden Ansprüche, dadurch **gekennzeichnet, daß** das Auslaßventil (6) und/oder das Einlaßventil (5) aus einem Ringeinsatz (7) und einer gegen die Stirnseite (8) dieses Ringeinsatzes (7) abdichtenden, einseitig angelenkten Klappe (9) bestehen.

8.  Atemvorrichtung nach Anspruch 7, dadurch **gekennzeichnet, daß** die Klappe (9) aus flexiblem, insbesondere gummielastischem Material besteht.

9.  Atemvorrichtung nach Anspruch 7 oder 8, dadurch **gekennzeichnet, daß** die Klappe (9) mittels eines oder mehrerer, insbesondere zweier Stifte (10) auf dem Ringeinsatz (7) befestigt ist.

10. Atemvorrichtung nach einem der Ansprüche 7 bis 9, dadurch **gekennzeichnet, daß** eine zusätzliche Dichtlippe (7) zwischen Ringeinsatz (7) und Klappe (9) vorgesehen ist.

11. Atemvorrichtung nach einem der Ansprüche 7 bis 10, dadurch **gekennzeichnet, daß** das Einlaßventil (5) und das Auslaßventil (6) innerhalb des Einlaßstutzens (1) bzw. Auslaßstutzens (2) klemmend befestigt sind, so daß sie austauschbar sind.

12. Atemvorrichtung nach einem der vorangehenden Ansprüche, dadurch **gekennzeichnet, daß** der Einlaßstutzen (1) an seinem freien Ende einen lösbaren Anschluß (14) für ein insbesondere als Aerosol-Vernebelungsgerät ausgebildetes medizinisches Gerät (15) aufweist.

13. Atemvorrichtung nach den Ansprüchen 7 und 12, dadurch **gekennzeichnet,** daß der Ringeinsatz (7) des Einlaßventils (5) und ein Rohrstück des medizinischen Gerätes (15) in das freie Ende des Einlaßstutzens (1) einsetzbar sind.

14. Atemvorrichtung nach einem der vorangehenden Ansprüche, dadurch **gekennzeichnet,** daß der Einlaßstutzen (1) und der Auslaßstutzen (2) gleichsinnig schräg in den Verbindungsstutzen (3) einmünden.

15. Atemvorrichtung nach Anspruch 14, dadurch **gekennzeichnet,** daß der Einlaßstutzen (1) und der Auslaßstutzen (2) einen spitzen Winkel einschließen.

16. Atemvorrichtung nach Anspruch 15, dadurch **gekennzeichnet,** daß der Winkel zwischen 40° und 70° liegt.

17. Atemvorrichtung nach einem der vorangehenden Ansprüche, dadurch **gekennzeichnet,** daß auf den Verbindungsstutzen (3) ein Mundstück (13) aufgesteckt ist.

18. Atemvorrichtung nach einem der vorangehenden Ansprüche, dadurch **gekennzeichnet,** daß eine verschließbare Öffnung (16) für den Anschluß einer Druckmeßvorrichtung vorgesehen ist.

## Claims

1. A respiratory device for monitored inhalation and exhalation for the purpose of respiratory duct therapy, comprising
   - an inlet pipe (1) for air to be inhaled,
   - an outlet pipe (2) for exhaled air,
   - a connection pipe (3) which is connected to the inlet pipe (1) and the outlet pipe (2),
   - an inlet valve (5) which is arranged in the inlet pipe (1), has the form of a one-way valve, and opens in the direction of the flow in the inlet pipe (1) and closes in the opposite direction,
   - an exhalation resistance (4) which is arranged on the outlet pipe (2) and against which the patient exhales and
   - an outlet valve (6) which is arranged in the flow path of the outlet pipe (2), has the form of a one-way valve, and opens in the direction of the flow in the outlet pipe (2) and closes in the opposite direction,
   - whereby inhalation of air through the exhalation resistance (4) and the outlet pipe (2) is prevented,
   characterised in that
   - the outlet valve (6) is arranged on the outlet pipe (2) and
   - the exhalation resistance has the form of a stepped tube (11), where the inner diameter of the outlet-end tube segment (12) is substantially smaller than that of the outlet pipe (2).

2. A respiratory device as claimed in Claim 1, characterised in that the exhalation resistance (4) comprises adjustable outlet openings.

3. A respiratory device as claimed in Claim 1 or 2, characterised in that the exhalation resistance (4) can be attached to the outlet pipe (2).

4. A respiratory device as claimed in one of the preceding claims, characterised in that the outlet valve (6) is inserted into the free end of the outlet pipe (2).

5. A respiratory device as claimed in one of the preceding claims, characterised in that the inlet valve (6) is inserted into the free end of the inlet pipe (1).

6. A respiratory device as claimed in one of the preceding claims, characterised in that the outlet valve (6) and/or the inlet valve (5) are detachable.

7. A respiratory device as claimed in one of the preceding claims, characterised in that the outlet valve (6) and/or the inlet valve (5) are composed of an annular insert (7) and a flap (9) which forms a seal with the end side (8) of this annular insert (7) and is articulated on one side.

8. A respiratory device as claimed in Claim 7, characterised in that the flap (9) is composed of flexible, in particular rubber-elastic material.

9. A respiratory device as claimed in Claim 7 or 8, characterised in that the flap (9) is fixed on the annular insert (7) by means of one or more, in particular two, pins (10).

10. A respiratory device as claimed in one of Claims 7 to 9, characterised in that an additional sealing lip (7) is arranged between the annular insert (7) and the flap (9).

11. A respiratory device as claimed in one of Claims 7 to 10, characterised in that the inlet valve (5) and the outlet valve (6) are attached by clamping inside the inlet pipe (1) and outlet pipe (2) respectively in such manner that they are exchangeable.

12. A respiratory device as claimed in one of the preceding claims, characterised in that at its free end the inlet pipe (1) comprises a detachable terminal (14) for a medical device (15) in particular in the form of an aerosol atomizer.

13. A respiratory device as claimed in Claims 7 and 12, characterised in that the annular insert (7) of the inlet valve (5) and a tubular segment of the medical device (15) can be inserted into the free end of the inlet pipe (1).

14. A respiratory device as claimed in one of the preceding claims, characterised in that the inlet pipe (1) and the outlet pipe (2) lead obliquely and in the same direction into the connection pipe (3).

15. A respiratory device as claimed in Claim 14, characterised in that the inlet pipe (1) and the outlet pipe (2) form an acute angle.

16. A respiratory device as claimed in Claim 15, characterised in that the angle amounts to between 40° and 70°.

17. A respiratory device as claimed in one of the preceding claims, characterised in that a mouth-piece (13) is attached to the connection pipe (3).

18. A respiratory device as claimed in one of the preceding claims, characterised in that a closeable opening (16) is provided for the connection of a pressure measuring device.

**Revendications**

1. Dispositif respiratoire pour produire une inhalation et exhalation contrôlée en vue de la thérapie des voies respiratoires, comprenant :
   - une tubulure d'admission (1) pour l'air à inhaler,
   - une tubulure d'échappement (2), pour l'air à exhaler,
   - une tubulure de liaison (3), reliée à la tubulure d'admission (1) et à la tubulure d'échappement (2),
   - une valve d'admission (5), prévue dans la tubulure d'admission (1) et réalisée sous forme de valve unidirectionnelle, qui s'ouvre dans le sens de l'écoulement allant dans la tubulure d'admission (1) et se ferme dans le sens inverse,
   - une résistance d'exhalation (4), disposée sur la tubulure d'échappement (2), contre l'action de laquelle le patient exhale, et
   - une valve d'échappement (6), disposée sur le chemin d'écoulement de la tubulure d'échappement (2) et réalisée sous forme de valve unidirectionnelle, qui s'ouvre dans la direction de l'écoulement allant dans la tubulure d'échappement (2) et se ferme dans le sens inverse,
   - faisant qu'est empêchée une inhalation d'air passant par la résistance d'exhalation (4) et la tubulure d'échappement (2), caractérisé en ce que
   - la valve d'échappement (6) est prévue sur la tubulure d'échappement (2),
   - la résistance d'exhalation (4) est réalisée sous forme de tube (11), à étagement allant en diminuant, le diamètre intérieur de la section de tube (12) située côté échappement étant sensiblement inférieur à celui de la tubulure d'échappement (2).

2. Dispositif respiratoire selon la revendication 1, caractérisé en ce que la résistance d'exhalation (4) présente des ouvertures d'échappement réglables.

3. Dispositif respiratoire selon la revendication 1 ou 2, caractérisé en ce que la résistance d'exhalation (4) peut être enfichée sur la tubulure d'échappement (2).

4. Dispositif respiratoire selon l'une des revendications précédentes, caractérisé en ce que la valve d'échappement (6) est insérée dans l'extrémité libre de la tubulure d'échappement (2).

5. Dispositif respiratoire selon l'une des revendications précédentes, caractérisé en ce que la valve d'admission (6) est insérée dans l'extrémité libre de la tubulure d'admission (1).

6. Dispositif respiratoire selon l'une des revendications précédentes, caractérisé en ce que la valve d'échappement (6) et/ou la valve d'admission (5) sont démontables.

7. Dispositif respiratoire selon l'une des revendications précédentes, caractérisé en ce que la valve d'échappement (6) et/ou la valve d'admission (5) sont composées d'une garniture annulaire (7) et d'un clapet (9), assurant l'étanchéité contre la face frontale (8) de cette garniture annulaire (7) et articulé sur un côté.

8. Dispositif respiratoire selon la revendication 7, caractérisé en ce que le clapet (9) est composé en un matériau flexible, en particulier ayant l'élasticité du caoutchouc.

9. Dispositif respiratoire selon la revendication 7 ou 8, caractérisé en ce que le clapet (9) est fixé sur la garniture annulaire (7) au moyen d'une ou plusieurs tiges (10), en particulier de deux tiges.

10. Dispositif respiratoire selon l'une des revendications 7 à 9, caractérisé en ce qu'une lèvre d'étanchéité supplémentaire (7) et prévue entre la garniture annulaire (7) et le clapet (9).

11. Dispositif respiratoire selon l'une des revendications 7 à 10, caractérisé en ce que la valve d'admission (5) et la valve d'échappement (6) sont fixées avec serrage à l'intérieur de la tubulure d'admission (1), respectivement de la tubulure d'échappement (2), de façon à être interchangeables individuellement.

12. Dispositif respiratoire selon l'une des revendications précédentes, caractérisé en ce que la tubulure d'admission (1) présente à son extrémité libre un raccordement (14) démontable, pour un appareil médical (15), réalisé en particulier sous forme d'appareil de nébulisation d'aérosol.

13. Dispositif respiratoire selon les revendications 7 et 12, caractérisé en ce que la garniture annulaire (7) de la valve d'admission (5) et une pièce tubulaire de l'appareil médical (15) sont susceptibles d'être enfichés dans l'extrémité libre de la tubulure d'admission (1).

14. Dispositif respiratoire selon l'une des revendications précédentes, caractérisé en ce que la tubulure d'admission (1) et la tubulure d'échappement (2) débouchent en biais, dans le même sens, dans la tubulure de liaison (3).

15. Dispositif respiratoire selon la revendication 14, caractérisé en ce que la tubulure d'admission (1) et la tubulure d'échappement (2) font un angle aigu.

16. Dispositif respiratoire selon la revendication 15, caractérisé en ce que l'angle est compris entre 40° et 70°.

17. Dispositif respiratoire selon l'une des revendications précédentes, caractérisé en ce qu'une pièce d'embouchure (13) est enfichée sur la tubulure de liaison (3).

18. Dispositif respiratoire selon l'une des revendications précédentes, caractérisé en ce qu'une ouverture (16) obturable est prévue pour le raccordement d'un dispositif de mesure de pression.

Fig.1

Fig.2